# EUROPEAN PATENT SPECIFICATION

(11) **EP 3 523 601 B1**
(45) Date of publication and mention of the grant of the patent: **18.05.2022**
(21) Application number: 16788806.4
(22) Date of filing: 05.10.2016
(51) Int. Cl.: G01B 11/02, A43D 1/02

(54) **FOOT SHAPE ACQUISITION USING DEPTH SENSOR AND PRESSURE PLATE TECHNOLOGY**
FUSSFORMERFASSUNG MIT TIEFENSENSOR UND DRUCKPLATTENTECHNOLOGIE
ACQUISITION DE FORME DE PIED À L'AIDE D'UNE TECHNOLOGIE DE CAPTEUR DE PROFONDEUR ET DE PLATEAU DE PRESSION

(43) Date of publication of application: 14.08.2019
(73) Proprietor: SafeSize Holding B.V., 1314 SK Almere (NL); UCS kupcu prilagojeni proizvodi, d.o.o., 1000 Ljubljana (SI)
(72) Inventor: LAHAJNAR, Leon, 5282 Cerkno (SI); OMRCEN, Damir, 1000 Ljubljana (SI); KOLSEK, Tomaz, 1000 Ljubljana (SI); STAVRAKIS, Angelos, 7442 DR Nijverdal (NL)
(74) Representative: V.O.
(86) International application number: PCT/IB2016/055961
(87) International publication number: WO 2018/065803

(56) References cited:
- WO-A1-2004/070315
- CN-A- 109 288 175
- DE-A1-102011 007 678
- DE-U1-202006 014 389
- JP-A- H11 101 623
- US-A- 5 689 446
- US-A- 5 790 256
- US-A1- 2006 098 896
- US-A1- 2006 104 503
- US-A1- 2009 247 909
- US-A1- 2014 285 646

## Description

### FIELD

The present disclosure relates to an apparatus for acquiring shape of one or two feet, and an associated method and use, and generally relates to foot measuring devices, IPC A61B5/1074 Foot measuring devices, A43D1/025 Foot or last measuring devices.

### BACKGROUND

This section provides background information, which is not necessarily prior art.

The traditional footwear industry and sales relies on the assumption that a shoe of particular length should fit a foot of a corresponding length. Therefore, shoes are traditionally manufactured in sizes with a particular step (6.67mm in EU sizing, 8.4mm for UK sizing, etc.).

It is also assumed that tolerances within manufacturing should not result in a considerable shoe length dispersion within a selected size. On the other hand, people with particular foot length (measured in mm) are assumed to select a particular size. However, it can be proved that both above assumptions are false to a large extent. Invalidity of these assumptions may have severe negative consequences in some businesses, such as internet shoe sales, where the end consumer does not have a chance of trying the individual sizes prior to purchase.

For proper fit, the accurate measurement of both shoes and feet is necessary. It has been proved, that although important, the length is not the only relevant characteristics of shoe and the foot. For a foot measurement, the process has to be made fast, reliable, accurate, easy to use and cheap. There are number of devices for foot measurement already available, based on various working principles. For example, these principles are:

Manual driven mechanical devices, originating from "Brannock" device, which usually have a static heel barrier, and a manual driven slider, which slides along measurement tape. The slider is pushed with a low force against human foot. Variants of this device can measure also width of the foot and sometimes the ball length.

Automated mechanical device, where pushing of sliders is implemented by means of electrical or pneumatic or other energy source, and stopping of a slider is implemented either by sensing the pressure against the foot or optically.

Laser based measurement devices, which use various laser projectors, which emit a particular light pattern, typically a line, and a camera, which records the emitted light. By knowing the position of both projector and a camera, it is possible to reconstruct the depth information and 3D coordinates of laser-illuminated area.

Ordinary light in combination with highly patterned socks. The images of deformed sock patterns can be analyzed to arrive at depth information.

In past years new affordable optical sensors have been developed, which contain both a light pattern emitter and the camera on the same printed circuit board (PCB). The emitter emits several different light patterns with a high frequency, whereas the camera captures these patterns. Vendor supplied software is able to reconstruct the "depth" of each pixel, hence the name "depth sensor" or "depth camera". Usually the applied light frequency is from infra-red spectrum, and the detection distance range can vary from 18cm to several meters. Examples of these sensors are Intel-RealSense, Occiptal-Structure, Microsoft-Kinect, Asus Xtion, etc.

Pressure plates have been in use for several years by podiatrists, clinical examination, sport shoe sales and biometric researchers. They can provide information on walking irregularities, pronation and supination, etc. Vendors supply pressure plates with analysis software with various utility analysis, such as calculation of pressure line, maximum value tracking, pressure versus time, etc.

Various applications require reliable and accurate foot scanners. For example, podiatrists measure humans feet to design and eventually manufacture custom made insoles, which fit a particular individual. Another example is application in running sport, where it is important that a particular shoe type is properly selected with respect to human feet shape, especially with respect to arch height. And an example of usage is in the shoe retail, where it is important to select a proper size of the shoe with respect to particular foot of the individual.

Every particular application may have different expectations from a foot scanner. It is assumed to be reliable, accurate, and preferably cheap. However, these requirements may not all be fulfilled to the very high level.

US2009/0247909 discloses a foot scanning apparatus, wherein on a foot rest two heel barriers are placed, against which a user has to position his or her heel. At a front side, spaced above the toe side of the feet, two spaced apart TV cameras are positioned, shown having parallel optical axes. The foot rest comprises pressure plates for measuring foot pressure. An image of each foot can be obtained by processing the pixels obtained from the cameras and data from the pressure plates.

DE202006014389 discloses a foot scanning apparatus, comprising a foot rest. On the foot rest a heel barrier is provided, against which a user has to position his or her heel. A stand is provided, extending vertically from a from side of the foot rest, i.e. from the toe side of the foot rest. A laser scanner is provided in the stand, for scanning the foot from the front from above the toe side, a CCD camera being provided in the stand for capturing the scanning lines obtained over the top of the foot. Images obtained from above the toe side of the feet can then be presented on a display.

US5689446 discloses an apparatus for determining a contour of the underside of a person's foot. A foot rest is provided bearing a heel barrier, against which a user has to position his or her heel. The heel barrier is provided at a rear end of a pressure plate comprising an array of gauge pins for obtaining a foot bed profile. In this apparatus the gauge pins are biased upward. A person places his or her foot on the gauge pins, such that these are pressed down according to the person's foot bed, and are then locked in position. After removal of the foot from the foot rest then an image is obtained of the profile represented by the gauge pins, using a projector and a camera placed on a stand at a toe end of the foot rest.

DE102011007678 discloses a foot imaging system, wherein a foot rest is provided with a heel barrier, against which a user has to position his or her heel. The foot rest is provided in an enclosed environment, shielded from outside light. A deck is provide above a forward, i.e. toe end side of the foot rest, carrying a optical camera for obtaining an image of a foot positioned on the foot rest against the heel barrier. As optical camera a stereo camera is proposed, or a camera with mirrors. The image obtained can be represented on a display.

All of these prior art apparatus need a heel barrier as a reference point for a foot in a foot rest, which influences the image of a foot and the measurements made with the apparatus. Moreover these prior art apparatus use either visual light or a laser scanner as a light source, and cameras for obtaining the image.

US2006/098896A1 discloses a method for establishing a position of an object to be scanned comprising the steps of: (i) placing the object on a scanning platform, wherein the scanning platform includes a predetermined scanning area having at least one sensor; (ii) obtaining a signal from the sensor; and (iii) analyzing the signal to determine if the object is within the predetermined scanning area.

JPH11101623A discloses a foot shape measuring device having measuring heads irradiating a foot with light and picking up optical images formed on the surface, a shape computing part for computing shape from picked-up image information and computing composite shape information of the foot through coordinate transformation and composition, and a control part.

US2014/285646A1 discloses a kiosk for the measurement of a plurality of characteristics of the foot, to compare the plurality of characteristics against a database of shoes, and to determine the best match between a shoe in the database and the measurement of the plurality of foot characteristics, the kiosk comprising: (a) a chamber with a closable door; (b) a transparent plate inside the chamber where the foot is placed for measurement; (c) a plurality of cameras in a variety of predetermined positions in the chamber directed toward the foot location to capture images of the foot such that a 3D model of the foot is generated from the images captured by the cameras; and (d) a database of shoes which includes the size and other measurements of each shoe, and a recommendation generator that generates recommendations of shoes based on relating the 3D model of the foot to the database of shoes.

US2006/104503A1 discloses an apparatus for reconstructing and measuring the three dimensional surface model of a foot rapidly, the apparatus comprising: a computer with display devices; a platform with frames to fasten a transparent plate; at least six image capture devices connected with the computer, installed on the platform, and implemented with geometric camera calibration; a thin and elastic sock printed with digitized codes that is worn on the foot of a person; a program, implemented in the computer, that could be used to: capture images of the sock worn on the foot; and reconstruct the 3D surface model and dimensions of the foot.

The present invention, as defined by the appended set of claims, provides a solution, which can offer reliable, accurate and low cost foot scanner for various fields of application, not limiting themselves to the ones presented above.

### BRIEF DESCRIPTION OF THE DRAWINGS

The drawings described herein are for illustrative purposes only and are not intended to define or limit the scope of the present disclosure, defined by the appended claims.

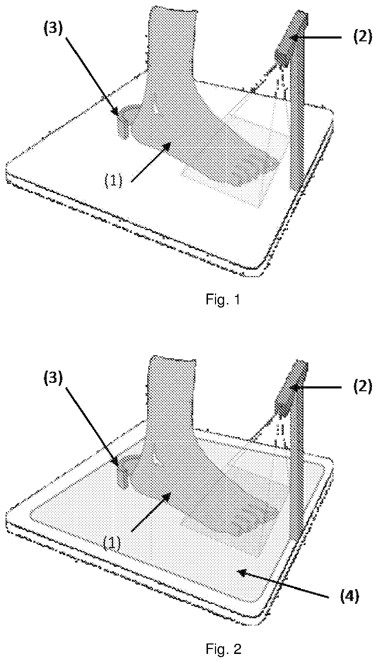

FIG.1 is showing an apparatus for measurement of feet shape not according to the claimed invention with a single depth sensor with the following elements: Feet (1), Depth Sensor (2), Heel Barrier (3);
FIG.2 is showing an apparatus for measurement of feet shape not according to the claimed invention with a single depth sensor in combination with pressure plate with the following elements: Feet (1), Depth Sensor (2), Heel Barrier (3), Pressure Plate (4);

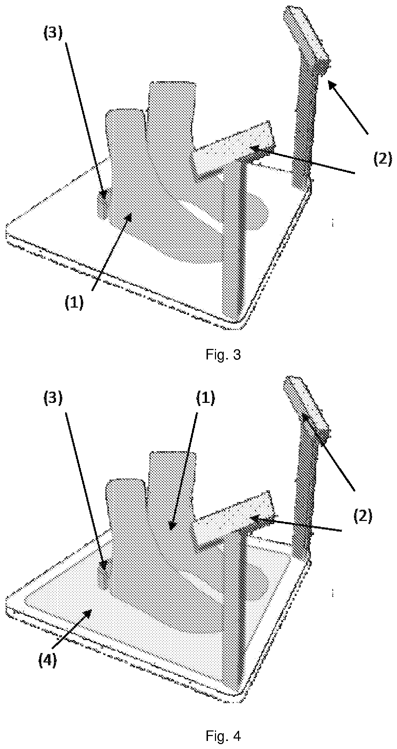

FIG.3 is showing an apparatus for measurement of feet shape not according to the claimed invention with two depth sensors with the following elements: Feet (1), Depth Sensors (2), Heel Barrier (3);
FIG.4 is showing an apparatus for measurement of feet shape not according to the claimed invention with two depth sensors in combination with pressure plate with the following elements: Feet (1), Depth Sensors (2), Heel Barrier (3), Pressure Plate (4);
FIG.5 is showing an apparatus for measurement of feet shape with three depth sensors with the following elements: Feet (1), Depth Sensors (2);
FIG.6 is showing an apparatus for measurement of feet shape with three depth sensors in combination with pressure plate with the following elements: Feet (1), Depth Sensors (2), Pressure Plate (4);
FIG.7 is showing an apparatus for measurement of feet shape with four depth sensors with the following elements: Feet (1), Depth Sensors (2);
FIG.8 is showing an apparatus for measurement of feet shape with four depth sensors in combination with pressure plate with the following elements: Feet (1), Depth Sensors (2), Pressure Plate (4); and
FIG.9 is showing a typical foot measurement apparatus together with processing and display devices with the following elements: Feet (1), Depth Sensors (2), Pressure Plate (4), Display Device (5), Processing Device (6), Mobile Device (7).

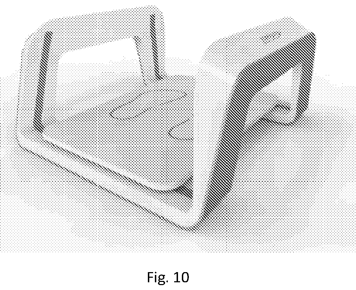

FIG.10 is showing the design of the commercial execution of the apparatus.

### DETAILED DESCRIPTION

Preferred embodiments of the invention are disclosed here, however, these embodiments are merely examples of the invention, which may be embodied in various forms.

The design and functions disclosed should not be interpreted as limiting, but as a representative to employ the invention in any appropriately detailed system.

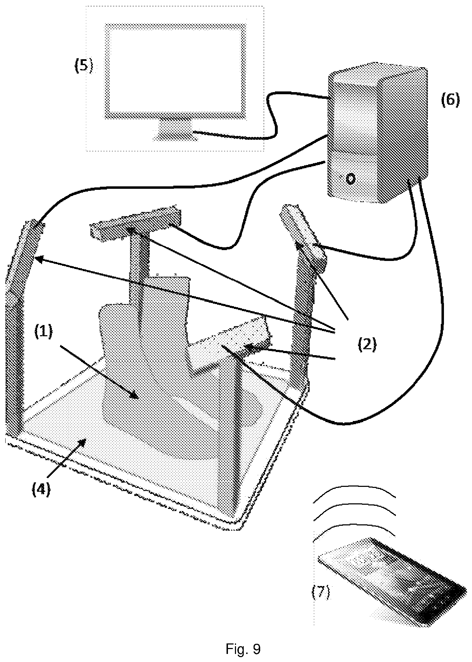

Fig. 9 is showing a typical foot measurement apparatus together with processing and display devices. Foot measurement apparatus consists of: a) mandatory depth sensor, b) optional pressure plate sensor, c) mandatory processing and control device, such as personal computer, d) optional display device, such as computer monitor or TV or a mobile tablet.

The process begins by end consumer taking off his shoes, rolling up his pants and stepping inside measurement area. Process continues by triggering the measurement on the processing and controlling device (PC). This starts measurement with depth sensors.

Depth sensors contain infra-red light pattern emitter, which drops several different light patterns with a high frequency onto the foot surface.

The emitted light is capture by a camera on the depth sensor. This results in a raster image of n x M pixels. The software provided by depth sensor vendor is capable of calculating the depth of each pixel by using triangulation method.

Each pixel with depth coordinate can be considered as a point in space. Points coming from a single sensor are called point clouds. Since sensors may pick up some noise, the point clouds have to be filtered by special software to eliminate as much noise as possible. Potential multiple point clouds coming from potential multiple sensors are combined to cover as much of foot surface as possible. To reduce the time for scanning, it is best to cover as much as possible of both feet surfaces in a single measurement. To be able to combine point clouds from multiple depth sensors, a transformation matrix has to be computed for each sensor. This can be achieved in multiple ways, for example by putting a rigid body of simple shape, which has a known geometry.

A point cloud may already suffice for certain applications, for example to extract some simple linear dimensions, such as length and width of the foot. If the surface coverage is good enough, it may be possible to extract partial or full silhouettes, for example side silhouette and top silhouette.

Point cloud may further be processed to create a triangulated surface. The advantages of such data form are easy and fast display of surface, ease of extraction of girths, less needed storage, etc.

The foot surface and extracted relevant dimensions are shown via graphical user interface to both end consumer or sales assistant. Data may also be stored in a central database for further usage.

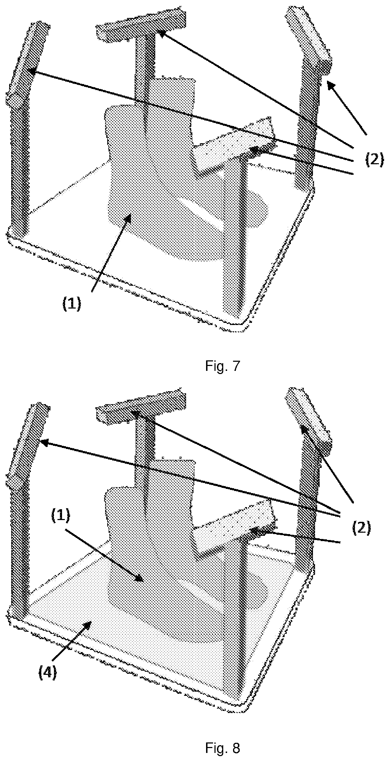

A complementary data can be received from a pressure plate sensor, which acquires pressure distribution between feet and the floor. A measurement apparatus can be formed, for example as depicted in Fig. 8, where end consumer can walk through apparatus, and pressure plate sensor dynamically records the pressure. By analyzing such data, it is possible to detect particular types of gait, pronation/supination, potential problems, etc.

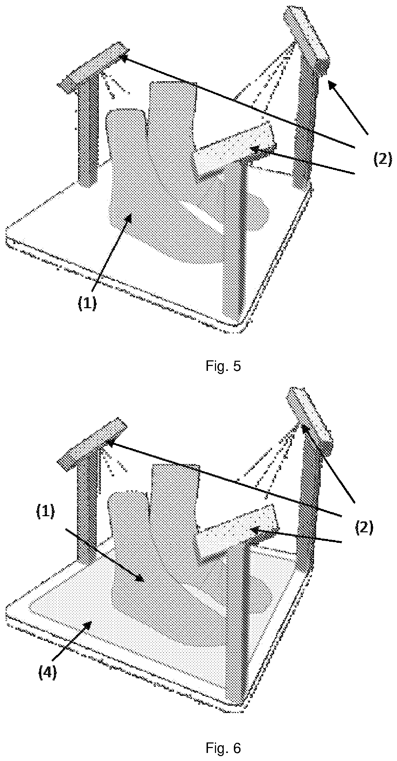

Figures 5-8 are showing possible implementations of the invention. With a measurement apparatus of the invention we get more complex solutions (Figures 5-8), which, result in higher reliability and accuracy.

### CITATIONS

US5128880A Foot measurement and footwear sizing system.
US5361133A Method and apparatus for analyzing feet.
US6289107B1 Apparatus and method of measuring human extremities using peripheral illumination techniques.
US6549639B1 Body part imaging system.
US3192627A Foot measuring and pedograph revealing machine.
US6834437B1 Foot measurement system.
US6160264A System for plotting a tri-dimensional shape, notably a plantar arch, and method of operating the system for producing an orthopaedic shoe or sole.
US8117922B2 Footcare product dispensing kiosk.
US20070253004A1 Foot Measuring Device.
US2942344A Foot measuring device.
US7421789B1 Systems and methods for footwear related measurement and adjustment.
EP0014022A1 Foot-size measuring apparatus.
US20030164954A1 Device for optoelectronically determining the length and/or the width of a body situated on a support.
US2200223A Foot measuring apparatus.
EP0760622B1 Digitised sensing process and arrangement for the three-dimensional shape in space of bodies or body parts.
US4164815A Device for measuring a human foot.
US5689446A Foot contour digitizer.
US3018554A Foot measuring device.
EP1418398A1 Shape measuring device.
US7516555B2 Systems and methods for footwear related measurement and adjustment. US20100286951A1 Foot measuring device.
US20100296726A1 High-resolution optical detection of the three-dimensional shape of bodies.
WO2004037085A1 Apparatus for determining size and shape of foot.
US20140182152A1 Footgauge.
DE102011007678A1 Measurement system for determining biometric data of human foot of children during shoe purchase, aligns human foot on boundary surface with respect to measurement coordinate system.
EP2954798A1 Foot scanner.
DE102010019234A1 Foot measuring device for e.g. human, for assisting individual during manufacture of shoe, has evaluation unit determining three-dimensional shape of part of foot from output signals of sensors i.e. ultrasonic sensors
DE102013001897A1 Method for determining biometric data of limb e.g. human foot, involves providing image acquisition generated by orientation sensor together with image analysis process data for determining measurement data and biometric data
US3931680A Foot measuring machines
US20030137510A1 Method and assembly for the photogrammetric detection of the 3-d shape of an object

## Claims

1. An apparatus for acquiring shape of one or two feet (1) comprising at least three depth sensors (2), wherein two depth sensors (2) are positioned above the front part of the foot (1) whereas one or two depth sensors (2) are positioned at the back of the foot (1) facing the heel, wherein the depth sensors (2) contain infra-red light pattern emitters.

2. An apparatus according to claim 1, **characterized by** using four depth sensors (2) to acquire shape of one or two feet (1), wherein two depth sensors (2) are positioned in the front of the foot (1) and two depth sensors (2) are positioned at the back of the foot (1).

3. An apparatus according to claim 1 or 2, **characterized by** five or more depth sensors (2) to acquire shape of one or two feet (1).

4. The apparatus according to any one of claims 1 - 3, wherein an additional pressure plate (4) is used, the apparatus being capable of acquisition of foot shape, as well as the static pressure distribution over human sole.

5. An apparatus according to any one of claims 1 - 4, wherein the apparatus is free of any heel barrier.

6. An apparatus according to any one of claims 1 - 5, wherein the apparatus further comprises a display device (5), a processing device (6) and a mobile device (7).

7. A method where information yielded from multiple depth sensors (2) is transformed into a unified coordinate system and a transformation is calculated for each sensor (2), wherein the data comes from multiple depth sensors (2) of an apparatus according to any one of claims 1 - 6, providing point clouds coming from multiple sources which are put into single coordinate system, wherein a rigid body with even surfaces and known angles between surfaces is used, an example of such body being a brick or a cube, to determine sensor locations in space, and thus put point clouds from different sources into a single coordinate system.

8. A method according to claim 7, wherein two steps are applied:
(10.1) application of filtering to each point cloud,
(10.2) application of filtering to combined point clouds.

9. A method according to claim 8, **characterized by**:
(11.1) extraction of linear dimensions directly by application of bounding box method, such as extracting foot length, foot width, etc.,
(11.2) extracting typical contours, such as side silhouette and top silhouette,
(11.3) apply point cloud triangulation algorithm, which results in an open or closed triangulated surface.

10. Method according to any one of claims 7 - 9, wherein a foot surface and relevant dimensions are shown via a graphical user interface, wherein data obtained is preferably stored in a central database for further usage.

11. Method according to at least claim 9, wherein the triangulated surface is used for extraction of foot characteristics.

12. Use of an apparatus according claims 1 - 6, for acquisition of foot shape, for example to extract foot length and foot width, and foot metatarsal girth.

## Patentansprüche

1. Einrichtung zum Erfassen der Form von einem oder zwei Füßen (1), umfassend wenigstens drei Tiefensensoren (2), wobei zwei Tiefensensoren (2) oberhalb des vorderen Teils des Fußes (1) positioniert sind, während ein oder zwei Tiefensensoren (2) an der der Ferse zugewandten Rückseite des Fußes (1) positioniert sind, wobei die Tiefensensoren (2) Infrarotlichtmuster-Emitter enthalten.

2. Einrichtung nach Anspruch 1, **gekennzeichnet durch** die Verwendung von vier Tiefensensoren (2) zur Erfassung der Form von einem oder zwei Füßen (1), wobei zwei Tiefensensoren (2) an der Vorderseite des Fußes (1) und zwei Tiefensensoren (2) an der Rückseite des Fußes (1) positioniert sind.

3. Einrichtung nach Anspruch 1 oder 2, **gekennzeichnet durch** fünf oder mehr Tiefensensoren (2) zur Erfassung der Form von einem oder zwei Füßen (1).

4. Einrichtung nach einem der Ansprüche 1 bis 3, wobei eine zusätzliche Druckplatte (4) verwendet wird, wobei die Einrichtung in der Lage ist, die Fußform sowie die statische Druckverteilung über die menschliche Sohle zu erfassen.

5. Einrichtung nach einem der Ansprüche 1 bis 4, wobei die Einrichtung frei von einer Fersenbarriere ist.

6. Einrichtung nach einem der Ansprüche 1 bis 5, wobei die Einrichtung ferner eine Anzeigevorrichtung (5), eine Verarbeitungsvorrichtung (6) und eine mobile Einrichtung (7) umfasst.

7. Verfahren bei dem von mehreren Tiefensensoren (2) gelieferte Informationen in ein einheitliches Koordinatensystem umgewandelt werden und für jeden Sensor (2) eine Transformation berechnet wird, wobei die Daten von mehreren Tiefensensoren (2) einer Einrichtung nach einem der Ansprüche 1 bis 6 stammen, wobei Punktwolken aus mehreren Quellen bereitgestellt werden, die in ein einziges Koordinatensystem eingebracht werden, wobei ein starrer Körper mit ebenen Oberflächen und bekannten Winkeln zwischen Oberflächen verwendet wird, wobei ein Beispiel für einen solchen Körper ein Ziegelstein oder ein Würfel ist, um Sensorpositionen im Raum zu bestimmen und somit Punktwolken aus verschiedenen Quellen in ein einziges Koordinatensystem zu bringen.

8. Verfahren nach Anspruch 7, wobei zwei Schritte angewendet werden:
(10.1) Anwendung der Filterung auf jede Punktwolke,
(10.2) Anwendung der Filterung auf kombinierte Punktwolken.

9. Verfahren nach Anspruch 8, **gekennzeichnet durch**:
(11.1) Extraktion linearer Abmessungen direkt durch Anwendung des Bounding-Box-Verfahrens, wie Extrahieren von Fußlänge, Fußbreite usw.
(11.2) Extrahieren typischer Konturen wie Seitensilhouette und Topsilhouette,
(11,3) Anwenden des Triangulationsalgorithmus für Punktwolken, der zu einer offenen oder geschlossenen dreieckigen Oberfläche führt.

10. Verfahren nach einem der Ansprüche 7 bis 9, wobei eine Fußoberfläche und relevante Abmessungen über eine graphische Benutzeroberfläche gezeigt werden, wobei erhaltene Daten vorzugsweise zur weiteren Verwendung in einer zentralen Datenbank gespeichert werden.

11. Verfahren nach wenigstens Anspruch 9, wobei die dreieckige Oberfläche zur Extraktion von Fußeigenschaften verwendet wird.

12. Verwendung einer Einrichtung nach Anspruch 1 bis 6, zur Erfassung der Fußform, zum Beispiel zur Extraktion von Fußlänge und Fußbreite und Mittelfußknochenumfang.

## Revendications

1. Appareil d'acquisition de la forme d'un ou de deux pieds (1) comprenant au moins trois capteurs de profondeur (2), dans lequel deux capteurs de profondeur (2) sont positionnés au-dessus de la partie avant du pied (1) tandis qu'un ou deux capteurs de profondeur (2) sont positionnés à l'arrière du pied (1) face au talon, les capteurs de profondeur (2) contenant des émetteurs de motifs lumineux infrarouges.

2. Appareil selon la revendication 1, **caractérisé par** l'utilisation de quatre capteurs de profondeur (2) pour acquérir la forme d'un ou de deux pieds (1), dans lequel deux capteurs de profondeur (2) sont positionnés à l'avant du pied (1) et deux capteurs de profondeur (2) sont positionnés à l'arrière du pied (1).

3. Appareil selon la revendication 1 ou 2, **caractérisé par** au moins cinq capteurs de profondeur (2) pour acquérir la forme d'un ou deux pieds (1).

4. Appareil selon l'une quelconque des revendications 1 à 3, dans lequel une plaque de pression supplémentaire (4) est utilisée, l'appareil étant capable d'acquérir la forme du pied, ainsi que la répartition de la pression statique sur la semelle humaine.

5. Appareil selon l'une quelconque des revendications 1 à 4, dans lequel l'appareil est exempt de toute barrière de talon.

6. Appareil selon l'une quelconque des revendications 1 à 5, dans lequel l'appareil comprend en outre un dispositif d'affichage (5), un dispositif de traitement (6) et un dispositif mobile (7).

7. Un procédé dans lequel les informations fournies par plusieurs capteurs de profondeur (2) sont transformées en un système de coordonnées unifié et une transformation est calculée pour chaque capteur (2), dans lequel les données proviennent de plusieurs capteurs de profondeur (2) d'un appareil selon l'un quelconque l'une des revendications 1 à 6, fournissant des nuages de points provenant de plusieurs sources qui sont mis dans un système de coordonnées unique, dans lequel un corps rigide avec des surfaces régulières et des angles connus entre les surfaces est utilisé, un exemple d'un tel corps étant une brique ou un cube, pour déterminer remplacements des capteurs dans l'espace, et ainsi mettre des nuages de points provenant de différentes sources dans un système de coordonnées unique.

8. Procédé selon la revendication 7, dans lequel deux étapes sont appliquées :
(10.1) l'application de filtrage à chaque nuage de points,
(10.2) l'application de filtrage aux nuages de points combinés.

9. Procédé selon la revendication 8, **caractérisé par** les étapes consistant à :
(11.1) extraire des dimensions linéaires directement par application de la méthode de la boîte englobante, telle que l'extraction de la longueur du pied, de la largeur du pied, etc.,
(11.2) extraire des contours typiques, tels que la silhouette latérale et la silhouette supérieure,
(11.3) appliquer un algorithme de triangulation de nuage de points, qui se traduit par une surface triangulée ouverte ou fermée.

10. Procédé selon l'une quelconque des revendications 7 à 9, dans lequel une surface de pied et des dimensions pertinentes sont affichées via une interface utilisateur graphique, dans lequel les données obtenues sont de préférence stockées dans une base de données centrale pour une utilisation ultérieure.

11. Procédé selon au moins la revendication 9, dans lequel la surface triangulée est utilisée pour l'extraction des caractéristiques du pied.

12. Utilisation d'un appareil selon les revendications 1 à 6, pour l'acquisition de la forme du pied, par exemple pour extraire la longueur et la largeur du pied, et la circonférence du métatarse du pied.
